# EUROPEAN PATENT APPLICATION

(11) **EP 2 730 294 A1**
(43) Date of publication of application: **14.05.2014**
(21) Application number: 12810747.1
(22) Date of filing: 06.04.2012
(51) Int. Cl.: A61L 9/16, A61L 9/00, B01D 53/06, B60H 3/00, F24F 7/00

(54) **AIR CLEANER**

(30) Priority: 08.07.2011 JP 2011151364
(71) Applicant: Mitsubishi Electric Corporation, Tokyo 100-8310 (JP); Mitsubishi Electric Home Appliance Co., Ltd., Fukaya-shi, Saitama 369-1295 (JP)
(72) Inventor: KUGE, Yousuke, Fukaya-shi Saitama 369-1295 (JP); AKARI, Yoshitaka, Fukaya-shi Saitama 369-1295 (JP); SATO, Akihisa, Tokyo 102-0073 (JP); AKAHORI, Katsuyuki, Fukaya-shi Saitama 369-1295 (JP); TOUDA, Yasumitsu, Fukaya-shi Saitama 369-1295 (JP)
(74) Representative: Pfenning, Meinig & Partner GbR
(86) International application number: PCT/JP2012/059451
(87) International publication number: WO 2013/008497

(57) **Abstract**

An air cleaner A includes: a portable casing 1 that includes therein an air trunk R having an inlet 11 and an outlet 42; blowing means 44 that is provided in the casing 1 and blows indoor air introduced from the inlet 11 into the air trunk R from the outlet 42; a deodorizing element 62 that is provided to interrupt the air trunk R, and through which the air introduced into the air trunk R passes; and control means 47 that controls an operation of the blowing means 44, wherein the deodorizing element 62 includes a catalyst that adsorbs an ammonia component in the air introduced into the air trunk R and collects the ammonia component from the air, and the control means 47 controls the blowing means 44 so that a flow rate of the air passing through the deodorizing element 62 is a predetermined value or less, and thus the deodorizing element collects at least 70% of the ammonia component in the indoor air introduced into the air trunk R.

## Description

### Technical Field

The present invention relates to an air cleaner that passes indoor air sucked into a body by a blower through a deodorizing portion to remove an odor component in the air to deodorize the indoor air.

### Background Art

Conventionally, there is an air cleaner including: a body case; an air inlet formed in a front surface of the body case; an air outlet formed in a rear of an upper surface of the body case; a fan provided in the body case for sucking air from the air inlet and blowing out the air to the air outlet; a fan motor for driving the fan; and a dust collection filter provided upstream of the fan for collecting dust in the sucked air, wherein a deodorizing portion formed of a catalyst attached to a surface of an adsorbent for adsorbing an odor component, and a heating portion for heating to recover a deodorizing function of the deodorizing portion are provided near the air outlet.

Such an air cleaner drives the fan to take indoor air from the air inlet into the body case, removes dust using the dust collection filter, and then deodorizes the indoor air by the odor component in the indoor air flowing down to the deodorizing portion being adsorbed by the adsorbent in the deodorizing potion.

The adsorbent in the deodorizing portion having adsorbed an odor is heated by the heating portion, and thus the odor component is removed therefrom. This can recover a deodorizing function of the adsorbent (for example, Patent Literature 1).

### Citation List

### Patent Literature

Patent Literature 1: Japanese Patent Laid-Open No. 2001-38126 (Figure 1)

### Summary of Invention

### Technical Problem

However, in the air cleaner described in Patent Literature 1, the deodorizing portion having the adsorbent for adsorbing an odor component is provided near the air outlet formed in the rear of the upper surface of the body case. Thus, the deodorizing portion needs to be placed in an extremely narrow limited region, and it is difficult to use an odor adsorbent having higher deodorizing performance and a larger deodorizing capacity.

Thus, with such a structure, even if the indoor air is passed through the deodorizing portion, it is difficult to significantly remove an odor at one time. Thus, the air cleaner needs to be operated for a long time until a person can feel a reduction in the odor in the indoor air.

Also, since the air outlet has a much smaller opening area than the air inlet, a flow rate of air flowing through the air outlet and the deodorizing portion provided near the air outlet is higher than a flow rate of air flowing near the air inlet.

Thus, with such a structure, air is discharged from the air cleaner into a room with the odor contained in the indoor air flowing through the deodorizing portion being insufficiently adsorbed by the adsorbent. Thus, the air cleaner needs to be operated for a long time until a person can feel a reduction in the odor in the indoor air.

Further, a heater that is the heating portion for heating the adsorbent to remove the odor adsorbed by the adsorbent is secured to the adsorbent, and thus there are parts with a short distance and a long distance between the heater and the adsorbent.

Thus, a part of the adsorbent close to the heater can be efficiently heated to remove an odor, while heat from the heater may be inefficiently transferred to a part of the adsorbent remote from the heater, and there is a possibility that an odor cannot be completely removed.

Thus, such a configuration cannot sufficiently recover an odor adsorbing capacity of the adsorbent, which may reduce a deodorizing function of the air cleaner.

The present invention is achieved to solve the above described problems, and has an object to provide an air cleaner that can efficiently reduce an odor in indoor air and can efficiently recover a function of an adsorbent that adsorbs an odor.

### Means for Solving the Problem

In accomplishing the above object, there is provided an air cleaner comprising: a portable casing that includes therein an air trunk having an inlet and an outlet;blowing means that is provided in the casing and blows indoor air introduced from the inlet into the air trunk from the outlet; a deodorizing element that is provided to interrupt the air trunk, and through which the air introduced into the air trunk passes; and control means that controls an operation of the blowing means, wherein the deodorizing element includes a catalyst that adsorbs an ammonia component in the air introduced into the air trunk and collects the ammonia component from the air, and the control means controls the blowing means so that a flow rate of the air passing through the deodorizing element is a predetermined value or less, and thus the deodorizing element collects at least 70% of the ammonia component in the indoor air introduced into the air trunk.

### Advantageous Effect of Invention

According to the present invention, an air cleaner can be provided having a quick deodorizing function capable of taking in more indoor air, and of efficiently reducing an odor in indoor air.

### Brief Description of Drawings

Figure 1(a) is a front view showing an air cleaner A according to Embodiment 1, Figure 1(b) is a top view showing the air cleaner A, and Figure 1(c) is a side view showing the air cleaner A.
Figure 2 is a front view (a), a top view (b) and a side view (c) showing a state in which a front panel, a prefilter and a HEPA filter were removed from an air cleaner A shown in Figure 1.
Figure 3 is an exploded perspective view of an air cleaner A shown in Figure 1.
Figure 4 is a Y-Y vertical cross-sectional view of an air cleaner A shown in Figure 1.
Figure 5(a) is a front perspective view of an air cleaner A shown in Figure 1, and Figure 5(b) is a rear perspective view of an air cleaner A shown in Figure 1.
Figure 6 is a perspective view showing a state in which a front panel, a prefilter and a HEPA filter were removed from an air cleaner A shown in Figure 5(a).
Figure 7 (a) is a front perspective view of a deodorizing portion of an air cleaner A according to Embodiment 1, and Figure 7(b) is a rear perspective view of the deodorizing portion.
Figure 8 is an exploded perspective view of a deodorizing portion of an air cleaner A shown in Figure 7(b).
Figure 9(a) is a plan view showing a back surface of a heating unit of an air cleaner A according to Embodiment 1, and Figure 9(b) is a Z-Z cross-sectional view of the heating unit.
Figure 10(a) is a perspective view showing a back surface of a heating unit shown in Figure 9, and Figure 10(b) is a perspective view showing a front surface of a heating unit shown in Figure 9.
Figure 11 is a rear exploded perspective view of a deodorizing portion of an air cleaner A according to Embodiment 2.
Figure 12(a) is a back plan view of a deodorizing portion of an air cleaner A according to Embodiment 2, and Figure 12(b) is an illustration showing a X-X cross-section of a deodorizing portion shown in Figure 12(a).
Figure 13(a) is a front perspective view of an air cleaner A according to Embodiment 3, and Figure 13(b) is a rear perspective view of the air cleaner A.
Figure 14 is rear perspective view showing a state in which a louver of an air cleaner A shown in Figure 13 is closed.
Figure 15 is a front view (a), a top view (b) and a side view (c) showing an air cleaner A according to Embodiment 4.
Figure 16(a) is a front perspective view of an air cleaner A shown in Figure 15, and Figure 16(b) is a rear perspective view of the air cleaner A.
Figure 17 is a bottom view of an air cleaner shown in Figure 15.
Figure 18 is a list of odor intensity in six grades corresponding to ammonia odor concentrations.
Figure 19 is a table of an indoor concentration and a blowing concentration of odor corresponding to an elapse time in Exemplary use of the air cleaner.

### Modes for Carrying Out the Invention

### (Embodiment 1)

Now, Embodiment 1 of the present invention will be described with reference to the drawings.

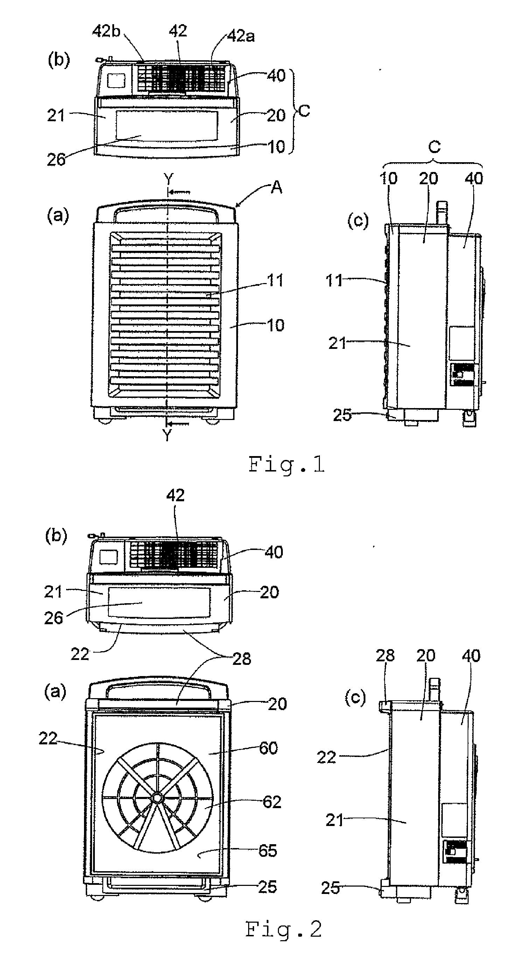

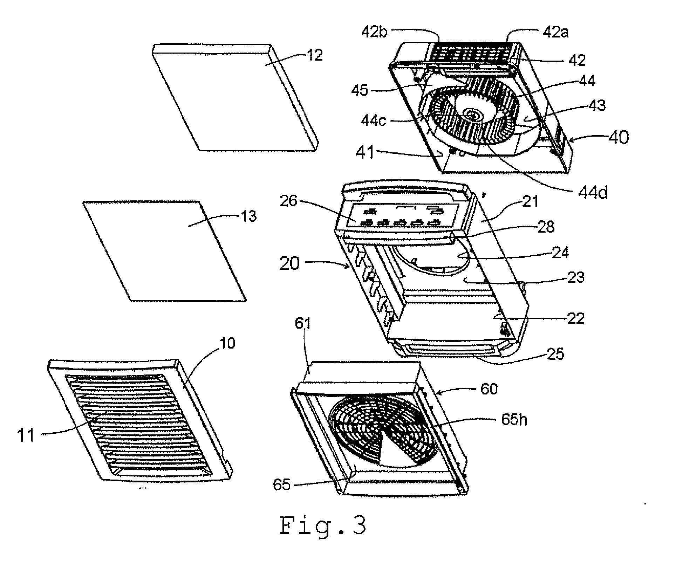

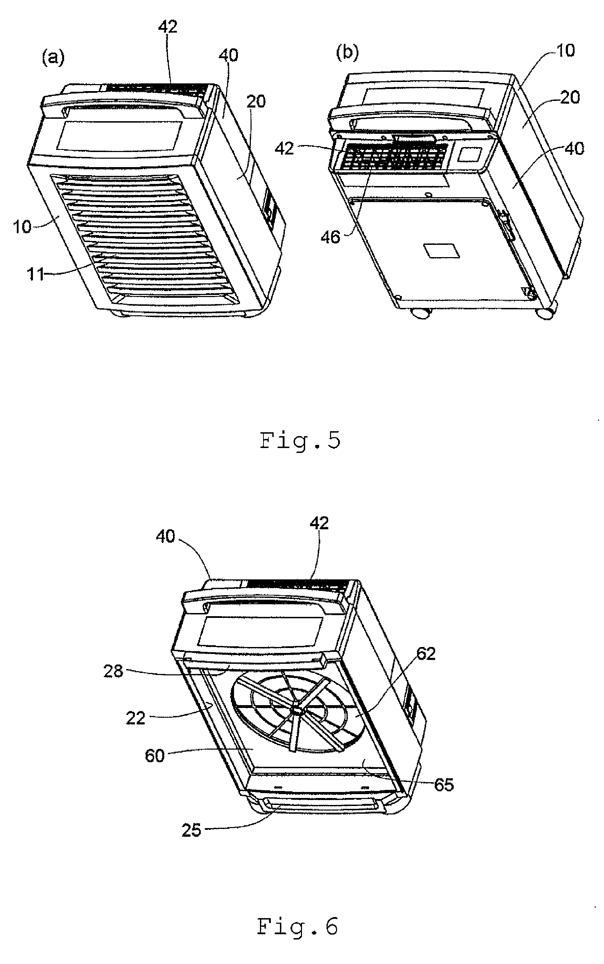

With reference to Figures 1 to 6, an air cleaner A according to this embodiment includes a body case C, which is a housing that forms an outer shell, and various functional components such as a deodorizing portion 60 provided in the body case C.

The body case C has a box shape made of resin, and includes a plurality of components such as a front panel 10, a front case 20, and a rear case 40.

The front case 20 includes, as a base, a frame 21 having a rectangular shape on front view and a predetermined depth. A rectangular front opening 22 is formed on a front side of the frame 21, and an opening on a rear side is covered with a partition plate 23.

A circular rear opening 24 that opens rearward is formed in the partition plate 23. Specifically, in the front case 20, the front opening 22 and the rear opening 24 communicate with each other. The rear opening 24 of the partition plate 23 forms a bell-mouth around a fan opening 44d of a blowing fan 44 described later.

Next, a lower side of the frame 21 of the front case 20 entirely protrudes forward beyond left and right sides to form a lower protrusion 25.

An upper side of the frame 21 protrudes forward beyond the left and right sides to form an upper protrusion 28, and an operating portion 26 including a plurality of operation buttons and an LED forming a display portion is provided on an upper front part of the upper side.

Correspondingly to the operating portion 26, an operating board (not shown) on which the operation buttons and the LED are mounted is provided on an upper inner part of the upper side of the frame 21. The operating board is electrically connected to a control means 47 described later.

Next, the front panel 10 has a rectangular shape on front view, and is configured to cover the front opening 22 of the front case 20 from the front.

Laterally extending slits are formed in a front surface of the front panel 10 to form an air inlet 11 (hereinafter, an inlet 11) that provides communication in a front-rear direction of the front panel 10. Specifically, the front panel 10 ensures ventilation so that air can flow through the front panel 10 in the front-rear direction.

Next, the rear case 40 has a rectangular shape on front view, has a front opening 41 on a front side and an opening to be an air outlet 42 (hereinafter, an outlet 42) in an upper surface, and is formed into a box shape with a closed rear surface 43.

On the rear surface 43, a blowing fan 44 as blowing means for taking indoor air into the air cleaner, and a partition 45 of a scroll shape that forms an air trunk for introducing air flowing down from the blowing fan 44 to the outlet 42 are provided.

The blowing fan 44 as the blowing means is connected to the control means 47, and the control means 47 controls rotation, stop, and the number of rotations of the blowing fan 44. As such, the control means 47 controls the blowing fan 44, and thus controls a blowing capacity thereof such as an air volume or a wind speed.

Also, in a space below the partition 45 formed by the rear case 40 and the partition 45, the control means 47 is provided that controls each component of the air cleaner A based on a predetermined program.

Further, a louver 46 that changes a direction of air blown from the outlet 42 into a room or closes the outlet 42 is provided near the outlet 42 in an upper part inside the rear case 40.

A grid is mounted to the opening of the outlet 42 so that the louver 46 is not directly touched.

Next, as the blowing fan 44, a multi-blade fan (sirocco fan) is used having multiple blades with a predetermined width in a rotational direction. Specifically, the blowing fan 44 includes multiple blades 44a located at a predetermined radius from a rotating shaft.

The blowing fan 44 is mounted to the rear surface 43 of the rear case 40. With the blowing fan 44 being mounted, a motor 44b that rotationally drives the blades 44a of the blowing fan 44 has a rotating shaft 44c directed forward and extending horizontally.

A fan opening 44d surrounded by the blades 44a opens forward.

The blowing fan 44 is thus mounted to the rear case 40, and thus the blowing fan 44 sucks air from the fan opening 44d directed forward in an axial direction of the rotating shaft 44c, and discharges the air radially of the blowing fan 44 including an upper part of the blowing fan 44.

Next, the partition plate 45 substantially vertically stands on the rear surface 43 of the rear case 40 so as to surround the blowing fan 44, one end thereof is connected to a right end 42a of the outlet 42, and the other end thereof is connected to a left end 42b of the outlet 42.

Specifically, the partition plate 45 is placed in the rear case 40 so as to surround the blowing fan 44 and form a bag shape by the ends being connected to the air outlet 42.

The louver 46 includes a plurality of plate-like wind direction plates 46a, a link mechanism 46c that connects the plurality of wind direction plates 46a and moves the wind direction plates 46a to a predetermined angle, and a drive portion (not shown) such as a motor that drives the link mechanism 46c.

In the louver 46, the plurality of plate-like wind direction plates 46a are arranged in parallel at predetermined intervals on the opening of the air outlet 42. Each wind direction plate 46a is pivotally supported on the outlet 42 by shafts 46d formed at opposite ends of the wind direction plate 46a.

A drive portion that drives the link mechanism 46c is connected to the control means 47 described later, and driven by a predetermined program depending on states of the air cleaner A, thereby changing a direction of the louver 46.

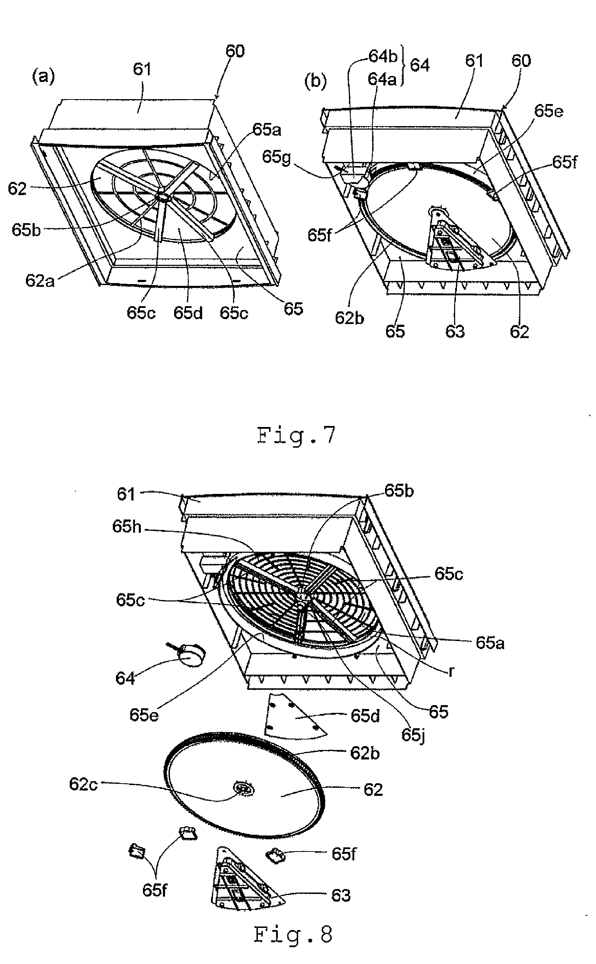

Next, with reference to Figures 7 and 8, a deodorizing portion 60 is a component through which indoor air taken into the air cleaner A is passed to remove an odor from the air. The deodorizing portion 60 includes a frame 61 as a base on which various components are provided, a deodorizing element 62, a heating unit 63 that locally heats the deodorizing element 62, and drive means 64 as position changing means for moving the deodorizing element 62 and changing a relative positional relationship between the heating unit 63 and a facing part of the deodorizing element.

First, the frame 61 has a rectangular shape on front view, and has a predetermined width. An outer shape of the frame 61 is sized to be fitted into the front opening 22 of the front case 20.

In the frame 61, an intermediate partition plate 65 is provided so as to block an opening of the frame 61 (so as to partition the opening into the front and rear).

A circular opening 65a that provides communication in the front-rear direction of the frame 61 is formed in the intermediate partition plate 65. A central support 65b is located at a center of the opening 65a, and a plurality of beams 65c are formed extending radially from the central support 65b to an opening edge of the opening 65a. The central support 65b has a shaft 65j protruding rearward.

Also, a frame 65h that allows air to flow into the opening 65a is provided on the front side of the opening 65a. The frame 65h prevents a user from directly touching the deodorizing element 62 described later.

Further, on a back surface (rear surface) of the intermediate partition plate 65, a ring-like guide portion 65e is formed standing rearward to surround the opening 65a.

A receiving portion 65f that receives the deodorizing element 62 described later when mounted to the guide portion 65e is provided on an edge of the guide portion 65e so as to protrude inward of the opening 65a.

The guide portion 65e is located on an outside of a circular opening edge of the circular opening 65a. Specifically, a gap r is formed between the edge of the opening 65a and the guide portion 65e. A diameter of the ring formed by the guide portion 65e is sized to hold therein the deodorizing element 62 including components described later.

A fan-like region that is located below the central support 65b of the opening 65a in the intermediate partition plate 65, and formed with a predetermined open angle equally extending to the left and right around the central support 65b is covered with a plate fan-like lid 65d.

The lid 65d is made of stainless, and secured to the beam 65c by a screw or the like from the rear side (back side) of the intermediate partition plate 65.

The lid 65d is placed to face the heating unit 63 described later, and sized to cover a heater unit 63a of the heating unit 63 when facing the heater unit 63a. Specifically, the heating unit 63 and the lid 65d face each other to form a heating space for the deodorizing element 62 described later.

The lid 65d is painted black to be heat resistant to increase thermal emissivity.

Next, the deodorizing element 62 includes a honeycomb core substrate having a disk planar shape, made of ceramic or aluminum, having a plurality of openings like honeycomb openings, and coated or impregnated with a catalyst by a binder.

The catalyst used is a catalyst having a property of adsorbing an odor (particularly, an odor of ammonia, methyl mercaptan, or hydrogen sulfide) such as a catalyst with manganese series (for example, a manganese deodorizing catalyst manufactured by SHINKO ACTEC CO.,LTD.) or platinum series.

Such a catalyst adsorbs an odor component, and then a catalytic action causes oxidation decomposition and decomposes the odor component into an odorless component. In particular, such a catalyst, when heated, provides a higher decomposition speed of the odor component.

An opening portion 62c is formed at a center of the deodorizing element 62, and an element frame 62a that is made of stainless and holds the deodorizing element 62 is provided on a front surface.

As described above, the deodorizing element 62 is formed of a honeycomb core, and the element frame 62a provided on the front surface has a predetermined opening. Thus, air can flow through the deodorizing element 62 in the fore/aft direction.

Further, a gear portion 62b is provided around the deodorizing element 62 in a peripheral edge of the deodorizing element 62.

A diameter of the deodorizing element 62 including the gear portion 62b is larger than a diameter of the circular opening 65a formed in the intermediate partition plate 65.

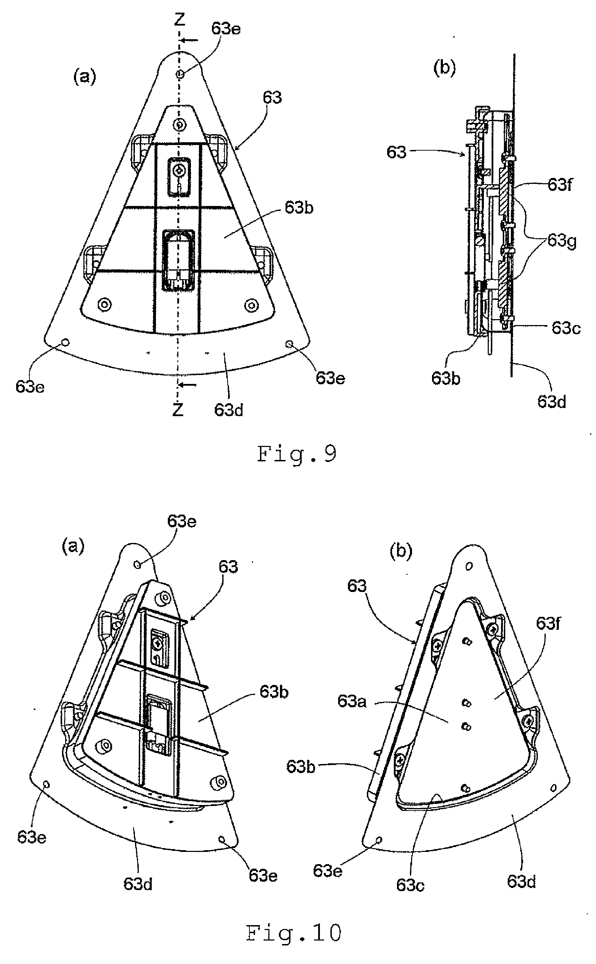

Next, with reference to Figures 9 and 10, the heating unit 63 includes a heater unit 63a as heating means for heating the deodorizing element 62, and a case 63b having a predetermined inner space that houses the heater unit 63a therein.

The heater unit 63a is electrically connected to the control means 47, and energization is controlled depending on states of the air cleaner A.

The heater unit 63a includes a plate-like heat generation portion 63f, and a heater portion 63g that heats the heat generation portion 63f. Further, the heat generation portion 63f has a fan-like planar shape, and a surface thereof is painted (black) to be heat resistant so as to increase emissivity of heat received from the heater portion 63f.

Specifically, in the heater unit 63a, the plate-like heat generation portion 63f receives the heat generated by the heater portion 63g, and the heat generation portion 63f radiates the heat from the entire plate surface, thereby heating the facing deodorizing element 62 with reduced irregularities.

The heater unit 63a is set to a heating capacity such that when the heater unit 63a is energized for a predetermined time, a part facing the deodorizing element 62 placed with a predetermined gap from the heater unit 63a can be heated to a predetermined temperature at which an odor adsorbed by the deodorizing element 62 can be removed. The deodorizing element 62 is thus heated to recover an odor collecting capacity.

The heating for recovering the odor collecting capacity is performed so that a temperature of a heated part of the deodorizing element 62 is about 120°C or more at which the deodorizing element 62 can decompose a collected odor component. The heating unit 63 is configured so as to reach the heating temperature within about 60 minutes.

As such, the heating step for recovering the odor collecting capacity can be performed within 60 minutes. Thus, a user can perform the step for recovering the odor collecting capacity of the deodorizing element 62 between operations using the air cleaner A or the like.

As the heater 63g, a PTC heater of semiconductor ceramic mainly consisting of barium titanate is used.

The PTC heater has self-temperature controllability, does not require external temperature control, and does not perform intermittent control as a thermostat. Thus, the PTC heater can be stably used without generation of spark or noise.

Next, the case 63b has a recess 63c that holds the heater unit 63a therein, and a flange portion 63d extending from an opening peripheral edge of the recess 63c.

The recess 63c has a fan shape matching the planar shape of the heater unit 63a, and the heater unit 63a is provided therein so as to face an opening of the recess 63c. The flange portion 63d has a screw hole 63e through which a screw is passed when the heating unit 63 is mounted to a predetermined position.

The heating unit 63 configured as described above has a fan-like planar shape matching a shape of a heat generation portion 63f of the heater unit 63a, and the opening of the recess 63c also has a fan shape.

Next, with reference to Figure 7, the drive means 64 is position changing means for moving the deodorizing element 62 and changing a relative positional relationship between the heating unit 63 and the facing part of the deodorizing element 62, that is, changing the part of the deodorizing element 62 facing the heating unit 63.

The drive means 64 includes a motor 64a, and a bracket 64b that holds the motor 64a. A gear is mounted to a rotating shaft of the motor 64a. The motor 64a is electrically connected to the control means 47, and energization is controlled depending on the states of the air cleaner A.

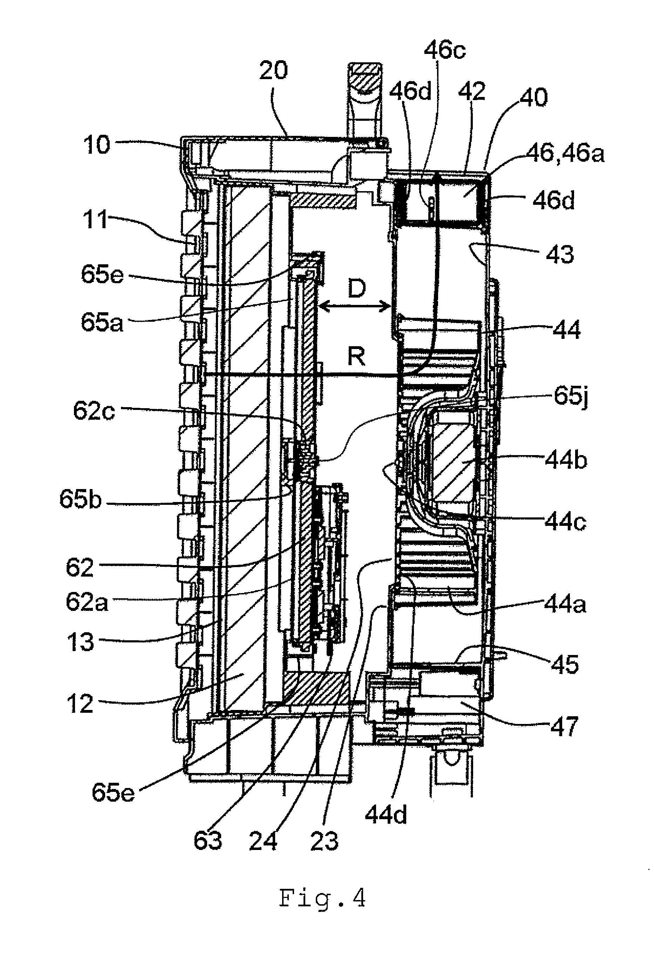

With reference to Figures 4, 7 and 8, the deodorizing element 62, the heating unit 63, and the drive means 64 are mounted to the frame 61 as described below to configure the deodorizing portion 60. In Figure 7, the frame 65h is removed for clear illustration of each component.

First, the opening portion 62c of the deodorizing element 62 as a bearing rotatably fits the shaft 65j provided on the central support 65b from a back side of the frame 61.

Thus, the deodorizing element 62 is rotatably placed in the frame 61 with the deodorizing element 62 facing the opening 65a inside the guide portion 65e formed on the back (rear) surface of the frame 61.

The receiving portion 65f that protrudes inward of the opening 65a is formed at the edge of the guide portion 65e. The receiving portion 65f receives the deodorizing element 62 and holds the deodorizing element 62 in the guide portion 65e from a rear (back) side so as not to significantly prevent movement of the deodorizing element 62 in a rotational direction.

The deodorizing element 62 is thus mounted to the frame 61, and the deodorizing element 62 is rotatably held to face the opening 65a in the intermediate partition plate 65 in a space surrounded by a back surface of the intermediate partition plate 65 (gap r), the guide portion 65e, and the receiving portion 65f.

The deodorizing element 62 may be held by the guide portion 65e rather than the shaft 65j provided on the central support 65b and the opening portion 62a of the deodorizing element 62 being configured as the shaft and the bearing as described above.

Then, with the deodorizing element 62 being placed on the frame 61 as described above, the heating unit 63 is mounted to the frame 61 so as to partially cover the deodorizing element 62 as described below.

First, the heating unit 63 is placed to span a part from the center to the lower side of the deodorizing element 62 so as not to prevent rotation of the deodorizing element 62.

In this state, the opening of the recess 63c provided with the heater unit 63a is directed forward so that the heater unit 63a of the heating unit 63 directly closely faces the deodorizing element 62.

The heating unit 63 is screwed to the central support 65b located in the opening portion 62c of the deodorizing element 62, and a mounting position formed in the intermediate partition plate 65 located outside the deodorizing element 62.

In this state, the heating unit 63 and the lid 65d face each other via the deodorizing element 62.

With the configuration as described above, the heating unit 63 is secured to the frame 61 so as not to prevent movement of the deodorizing element 62 in the rotational direction.

The heating unit 63 and the lid 65d face each other and are placed in the frame 61, and thus a space storing heat from the heater unit 63a is formed with the deodorizing element 62 placed between the heating unit 63 and the lid 65d. Further, the heat generation portion 63f is painted to increase heat emissivity, and thus efficiently radiates heat received from the heater portion 63g.

Thus, the heating unit 63 is configured to efficiently locally heat the part of the facing deodorizing element 62.

Next, in the drive means 64, a bracket 64b is secured to the intermediate partition plate 65 with the motor 64a being held by the bracket 64b. At this time, the gear mounted to the rotating shaft of the motor 64a meshes with the gear 62b provided on the deodorizing element 62.

The drive means 64 is placed between the opening 65a and a corner 65g of the intermediate partition plate 65 on the back surface of the intermediate partition plate 65. Further, the drive means 64 is preferably provided in a part between a corner 65g located in an upper part remote from the heating unit 63 among four corners 65g and the opening 65a.

The drive means 64 is thus provided, and energization control by the control means 47 drives the motor 64a to allow the deodorizing element 62 to be rotated with respect to the frame 61, and allow a part of the deodorizing element 62 facing the heating unit 63 to be changed.

Specifically, a relative positional relationship between the heating unit 63 and the deodorizing element 62 can be changed.

The drive portion 64 is provided between the opening 65a and the corner 65g, and thus a dead space around the opening 65a formed in the rectangular intermediate partition plate 65 can be effectively used. Further, the drive means 64 is provided in a position remote from the heating unit 63, and thus the drive portion 64 is insulated from the influence of heat from the heating unit 63.

Next, the front panel 10, the front case 20, the rear case 40, and the deodorizing portion 60 thus configured are assembled with other functional components as described below to configure the air cleaner A.

First, the rear case 40 is mounted to the rear surface of the front case 20 with the opening 41 being directed forward. At this time, the opening 44d of the blowing fan 44 provided in the rear case 40 faces the rear opening 24 formed in the partition plate 23 provided in the front case 20. The center of the rear opening 24 matches the axis of the rotating shaft of the blowing fan 44 in the front-rear direction.

Then, the deodorizing portion 60 is mounted to the front case 20 in such a manner that the frame 61 is inserted into the front case 20 from the front opening 22 of the front case 20, and an outer periphery of the frame 61 is held in the front case 20.

As such, with the deodorizing portion 60 being mounted to the front case 20, the rear side of the deodorizing portion 60 (side on which the heating unit 63 is mounted) is placed toward the rear opening 24 of the front case 20, and the heating unit 63 is located between the deodorizing element 62 and the rear opening 24.

The deodorizing element 62 faces the partition plate 23 and the rear opening 24 of the front case 20 that forms a bell-mouth around the opening 44d of the blowing fan 44 with a predetermined space D therebetween so as not to prevent an airflow from the deodorizing element 62 to the rear opening 24.

The heating unit 63 is located in the space D thus formed.

Then, a HEPA filter 12 of substantially the same size as the opening of the frame 61 is provided in the frame 61 of the deodorizing portion 60 mounted to the front case 20, and a prefilter 13 is provided to cover a front surface of the HEPA filter 12.

Then, the front panel 10 is provided between the upper protrusion 28 and the lower protrusion 25 on the front case 20 on the front side of the prefilter 13 to configure the air cleaner A.

The HEPA filter 12 is a filter for removing fine dust such as pollen, mite feces, mold spore, and house dust contained in air.

The prefilter 13 is a coarse filter for previously removing large dust contained in air before the HEPA filter filters the air, and for maintaining an effect of the HEPA filter for a long period.

Next, with reference to Figure 4, in the air cleaner A configured as described above, an air trunk R is formed from the inlet 11 to the outlet 42 that takes in indoor air, cleans and deodorizes the air, and discharges the air into a room. The air trunk R will be described with an air cleaning operation state of the air cleaner A and a flow of air taken in.

First, a user operates the operating portion 26 to input to the control means 47, and thus a predetermined program for operating the air cleaner A is executed.

When the blowing fan 44 is driven after the operation start described above, a suction force is generated for taking indoor air from the air inlet 11 into the air cleaner A, and the indoor air flows into the inlet 11.

The air taken in from the inlet 11 flows rearward in the air cleaner A, large dust is removed from the air by the prefilter 13, and then fine dust is removed by the HEPA filter 12.

Then, the air from which dust is removed flows further rearward and reaches the deodorizing portion 60, passes through the opening 65a, and reaches the deodorizing element 62 placed to face the opening portion 65a. The deodorizing element 62 is provided to interrupt the air trunk R, and has many openings in the form of a honeycomb from the front surface to the back surface, and a catalyst that adsorbs an odor is applied to the front surface.

Thus, air containing an odor passes through the openings in the form of a honeycomb when passing from the front side to the back side of the deodorizing element 62, and the catalyst applied to the deodorizing element 62 adsorbs the odor contained in the air, thereby removing the odor from the air.

"Removing the odor from the air" includes a state where the odor is completely removed from the air, and also a state where the odor is reduced from the state of the air before passing through the deodorizing element 62.

The air cleaner A is continuously operated as described above, and thus adsorbed odors are accumulated in the deodorizing element 62. With increasing adsorbed odors, a deodorizing capacity of the deodorizing element 62 is reduced.

Then, the air from which the dust and the odor are removed flows further rearward from the deodorizing element 62, passes through the rear opening 24 that opens in the partition plate 23 of the front case 20, and flows to the blowing fan 44 placed to face the rear opening 24.

The air flowing to the blowing fan 44 flows from a front side in an axial direction of the blowing fan 44 into the fan opening 44d surrounded by the blades 44a, and is discharged to the outside of the blowing fan 44 radially of the blowing fan 44 including the upper part of the blowing fan 44.

The air discharged from the blowing fan 44 is guided to the rear case 40 by the partition plate 45, adjusted in wind direction when passing through the louver 46, and blown out as cleaned air through the outlet 42 upward of the air cleaner A from inside the air cleaner A.

Thus, the air trunk R horizontally connects to a rear portion of the air cleaner body from the inlet 11, turns upward at the rear portion and reaches the outlet 42.

Specifically, in the air trunk R, in terms of the airflow, dust filtration filters such as the prefilter 13 and the HEPA filter 12 are placed upstream of the deodorizing element 62, and a bent portion at which the direction of the airflow is changed to be bent upward is formed downstream of the deodorizing element 62. A sirocco fan as the blowing fan 44 is located in the bent portion. The sirocco fan takes in air from the direction of the rotating shaft of the fan, and discharges the taken air radially of the fan. This forms a linear flow of the indoor air rearward from the front side of the body case C, and can efficiently change the wind direction toward the outlet 42.

The deodorizing element 62, the prefilter 13, the HEPA filter 12, and the front surface of the opening 44d of the blowing fan 44 are placed perpendicularly to the direction of air flowing through the air trunk R.

Thus, the air flows straight to the deodorizing element, and the air hits perpendicularly to each filter surface, thereby providing a good flow of air.

The opening 65a is located at a vertical center on the front side of the body case C, and a relationship between a projected area X of the body case C on front view and an area Y of the opening 65a on front view is "Y≥0.6X".

Next, for the deodorizing element 62 that has adsorbed many odors and been reduced in deodorizing performance through an air cleaning operation (deodorizing operation) as described above, an operation for recovering the deodorizing performance of the deodorizing element 62 will be described.

The control means 47 performs an operation for recovering the deodorizing performance as described below at predetermined timing, for example, when a cumulative operation time from an operation start or a previous operation for recovering the deodorizing performance of the deodorizing element 62 exceeds a predetermined time.

In a state where the blowing fan 44 stops, that is, after the air cleaner A finishes the air cleaning operation, or in a state where the air cleaner A does not perform the air cleaning operation, the control means 47 energizes the heater unit 63a to cause the heater unit 63a to generate heat, and heat the part of the deodorizing element 62 facing the heater unit 63a to a predetermined temperature for a predetermined time.

The heating temperature and the heating time of the deodorizing element 62 are sufficient for removing the odor adsorbed by the deodorizing element 62.

For the heated part of the deodorizing element 62, the front side is covered with the lid 63d painted to increase heat emissivity, and the rear side is covered with the heating unit 63 (heater unit 63a). Thus, heat generated from the heater unit 63a and heat radiated from the lid 63d can efficiently heat the deodorizing element 62.

The deodorizing element 62 is located between the heater unit 63a and the lid 63d, and thus heat radiated from these members is easily stored near the deodorizing element 62, thereby more efficiently heating the deodorizing element 62.

Next, as described above, when the operation for recovering the deodorizing performance of the part facing the heating unit 63 is finished, the control means 47 operates the drive means 64 for rotating the deodorizing unit 62 to rotate the deodorizing unit 62 by a predetermined angle.

By this operation, the part of the deodorizing element 62 facing the heating unit 63 and having been heated is offset in the rotational direction with respect to the heating element 63.

Thus, the part of the deodorizing unit 62 having been heated is moved out of the position between the heating unit 63 and the lid 63d, and a part of the deodorizing element 62 having adsorbed many odors is newly located between the heating unit 63 and the lid 63d.

The angle of rotation of the deodorizing unit 62 is preferably the same as or smaller than the open angle of the fan-like heater unit 63a. The rotation angle is thus set, and during one turn of the deodorizing unit 62, all parts of the deodorizing unit 62 stay in front of the heater unit 63a and are heated.

The deodorizing unit 62 may be moved immediately after heating, or immediately before a next air cleaning operation.

The components of the air cleaner A are configured as described above to obtain the advantages as described below.

As in this embodiment, the relative positional relationship between the deodorizing element 62 and the heating unit 63 that locally heats the deodorizing element 62 can be changed to reduce a size of the heating unit 63.

The reduction in size of the heating unit 63 has an advantage that, for example, there is no need to place a large heater that faces the all parts of the deodorizing element 62 to reliably heat the all parts of the deodorizing element 62, thereby simplifying the structure and reducing cost.

The relative positional relationship between the heating unit 63 and the deodorizing element 62 can be changed. Thus, when the all parts of the deodorizing element 62 are to be deodorized, the part of the deodorizing element 62 facing the heating unit 63 may be changed, and the heating unit 63 does not need to cover the all parts of the deodorizing element 62.

Specifically, the part of the deodorizing element 62 facing the heating unit 63 may be always limited, and thus the heating unit 63 minimally prevents the flow of air through the deodorizing element 62. Thus, more air can flow through the deodorizing element 62, and more odors can be removed from the air at a time.

Further, the relative positional relationship between the deodorizing element 62 and the heating unit 63 can be changed, and thus the heating unit 63 can reliably face and heat each part of the deodorizing element 62.

This can reduce irregular heating between the parts of the deodorizing element 62, and efficiently recover the deodorizing capacity of the deodorizing element 62.

Next, the inlet 11 of the air cleaner A is formed in the front surface of the body, and the outlet 42 is formed in either a side surface, a top surface, or a back surface of the body.

With such a configuration, the inlet 11 opening wide can easily face a source of an odor, and thus can more quickly suck the odor and remove the odor from the indoor air.

The outlet 11 is formed in either the side surface, the top surface, or the back surface of the body. Thus, cleaned air hardly flows to the source of the odor and diffusion of the odor can be prevented.

Next, the deodorizing element 62 is rotatably supported in the body case C, and the heating unit 63 is secured to the body case C close to the surface of the deodorizing element 62.

Thus, the heating unit 63 that causes the heater unit 63a to generate heat is secured in the body case C, and there is no need to consider routing of a wire for supplying power to generate heat and countermeasures against heat in a wide range in the body case C due to a positional change of a hot part in the body case C.

Also, the deodorizing element 62 is rotated to change the part facing the heating unit 63, and thus the deodorizing element 62 may be simply moved in one direction to cause the all parts of the deodorizing element 62 to face the heating unit 63 without irregularities.

Next, the deodorizing element 62 has a disk shape, and thus a rotation region of the deodorizing element 62 can be minimized with respect to an area of the deodorizing element 62 seen in the direction of the rotating shaft.

Specifically, the placement region of the deodorizing element 62 in the body case C can be minimized.

The deodorizing element 62 is rotated to change the part facing the heating unit 63. Thus, when the deodorizing element 62 has a disk shape, the heater unit 63a of the heating unit 63 having a size in a diametrical direction of the deodorizing element 62 at least equal to or smaller than a rotation radius of the deodorizing element 62 can heat many regions of the deodorizing element 62.

Further, the deodorizing element 62 has a disk shape, and thus has a configuration for achieving the above described advantages. Also, the circular shape can form a maximum region for an opening area of the body case C as the rectangular opening, and thus a larger region allowing deodorization can be formed.

Thus, more air can flow through the deodorizing element 62, thereby increasing an air volume while maintaining the deodorizing capacity.

Next, the surface of the deodorizing element 62 is coated or impregnated with a catalyst having an ammonia decomposition function. Thus, an air cleaner can be configured that can quickly deodorize pet odors or odors in nursing care in hospitals, care facilities, and care sites.

In particular, in this embodiment, odors can be removed from more air in a short time, thereby solving a problem of odors in nursing care in a place used by many people such as hospitals or care facilities.

Next, the blowing fan 44 as blowing means is located in the air trunk, the deodorizing element 62 is located upstream of the blowing fan 44 in the air trunk R, and the heating unit 63 is located between the blowing fan 44 and the deodorizing element 62.

With such a configuration, a space provided for reducing pressure loss (loss of an airflow) caused around the deodorizing element 62 and the opening 44d of the blowing fan 44 can be used to place the heating unit 63.

Next, the heating unit 63 includes the case 63b having an opening on a side facing the deodorizing element 62 and having a predetermined inner space, and the electrical heater unit 63a that is located in the inner space of the case 63b and radiates heat through the opening. The heater unit 63a is set to a heating capacity such that when the heater unit 63a is energized for a predetermined time, the facing part of the deodorizing element 62 can be heated to a predetermined temperature (for example, 150°C) within a predetermined time (for example, 30 minutes). Thus, the odors adsorbed by the deodorizing element 62 can be removed.

The case 63b has a fan shape, and thus can cover the deodorizing element 62 in a minimum area. The open angle of the fan shape is set with reference to a rotation angle of one turn of the deodorizing element 62.

Next, the control means 47 includes the control program for driving the drive means 64 as the position changing means at predetermined timing and rotating the deodorizing element 62.

Thus, when the deodorizing element 62 is heated, the control means 47 can automatically rotate the deodorizing element 62 and cause a part to be deodorized to face the heating unit 63. Specifically, when the control means 47 heats the deodorizing element 62, the heating unit 63 as the heating means is energized for a predetermined time while facing a predetermined region of the deodorizing element 62 to remove an odor adsorbed by the deodorizing element 62, thereby recovering a local odor collecting capacity of the deodorizing element 62.

The above described control program has a process step of driving the drive means 64 at predetermined timing, rotating the deodorizing element by a predetermined rotation angle, then stopping the deodorizing element, and energizing the heating unit for a predetermined time in the stop state.

Thus, a series of operations from the rotation to the heating of the deodorizing element 62 can be automatically performed by the control means 47.

The control to remove the odor adsorbed by the deodorizing element 62 is executed after the operation of the blowing fan 44 is stopped when a cumulative operation time or a cumulative number of operations of the blowing fan 44 as the blowing means exceeds a predetermined value.

Specifically, the control means 47 repeats the operation depending on the frequency of use of the air cleaner A, and sequentially changes the relative positional relationship between the deodorizing element 62 and the heating unit 63 in a predetermined direction.

This can maintain the odor collecting force of the entire deodorizing element 62.

Next, in the air trunk R, the dust filtration filters 12, 13 are placed upstream of the deodorizing element 62, the front panel 10 as a frame having ventilation is removably mounted to the front side of the body case C. With the frame being removed from the body case C, the dust filtration filters can be taken forward of the body case C.

With such a configuration, the dust filtration filter can be removed from the front side of the body case C, thereby increasing a maintenance property of the dust filtration filters to which large dust tends to adhere.

Next, the air trunk R is bent upward on a downstream side of the deodorizing element 62, and the blowing fan 44 is placed in the bent portion. The blowing fan 44 is a multi-blade fan that rotates around the rotating shaft extending horizontally, and also feeds air upward, which is introduced from the front side of the body case C.

The multi-blade fan (sirocco fan) takes in air from the direction of the rotating shaft of the fan, and discharges the taken air radially of the fan. This forms a linear flow of the indoor air rearward from the front side of the body case C, and can efficiently change the wind direction toward the outlet 42.

Next, the heating unit 63 is mounted to the body below the center of rotation of the deodorizing element 62.

As such, the heating unit 63 including the heater unit 63a or the like and having a certain level of weight is placed in a lower position to lower the center of gravity of the air cleaner A. Thus, the air cleaner A that can be stably placed on a floor surface can be configured.

Besides, the opening 65a is located at the vertical center on the front side of the body case C, and a relationship between a projected area A of the body case C on front view and an area B of the inlet on front view is "B≥0.6A".

This relationship allows the opening 65a and the inlet to take in indoor air to a maximum with respect to the area of the body case C on front view, and the air cleaner that can take in and pass more indoor air to the deodorizing element 62 can be configured.

Next, the air cleaner A configured as described above has a portable casing that is accessible and movable to an odor source with a configuration as described below.

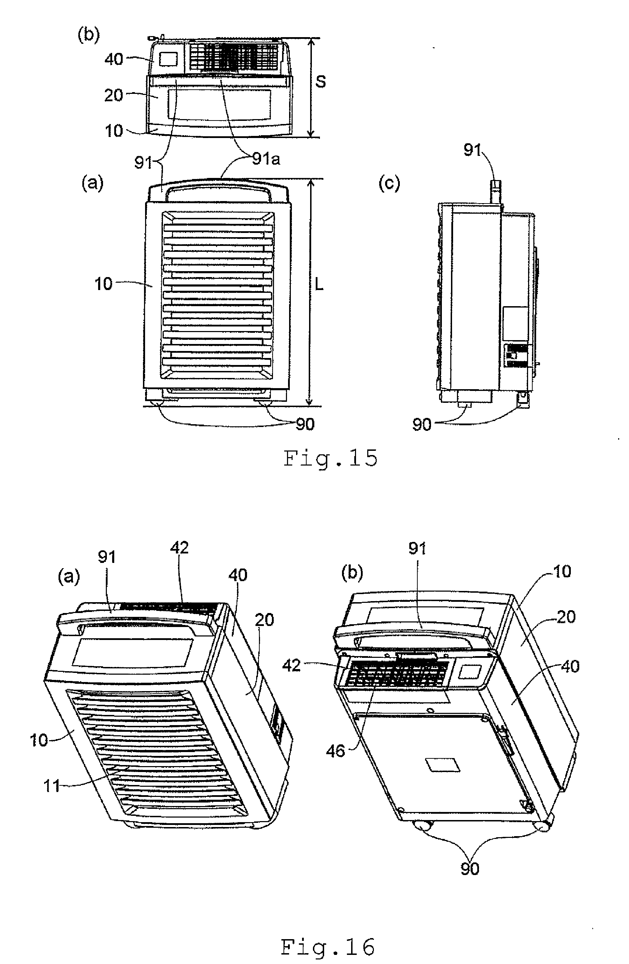

With reference to Figures 15 to 17, the air cleaner A includes the body case C as the outer shell and various functional components such as the deodorizing portion 60 provided in the body case C.

The body case C is made of resin, includes multiple components such as the front panel 10, the front case 20, and the rear case 40, and front and side planar shapes form a vertically rectangular outer shell.

Then, four wheels 90 are provided on a bottom of the body case C. Among the four wheels, two are provided on a bottom of the front case 20, and two are provided on a bottom of the rear case 40. When the body case C is seen from the front, one wheel is placed on the left and one wheel is placed on the right so as to be symmetrical with respect to the center of the body case C.

On the upper rear part of the upper side of the frame 21 that constitutes the front case 20, a handle 91 is provided gripped by a user when moving the air cleaner A.

The handle 91 is placed so that a central axis of a grip 91a is laterally placed at the center of the top surface of the body case C. The handle 91 is located between the heating unit 63 and the blowing fan 44 in the front-rear direction of the body case C. A height L of the grip 91a from the floor surface is 700 mm or less.

With the components being thus placed, the wheels 90 are configured to be rotatable at least longitudinally of the grip 91a.

As described above, the wheels are provided on the body case C so as to be rotatable longitudinally of the grip 91a of the handle 91, thereby allowing even a large air cleaner A to be easily pushed and pulled.

In particular, the handle 91 is located between the heating unit 63 and the blowing fan 44 in the front-rear direction of the body case C, and thus the handle 91 is located substantially between parts with high weight density. Thus, when the handle 91 is gripped to push and pull the air cleaner A, the air cleaner A can be moved at a position having relatively good balance of weight.

This provides the air cleaner A easily carried by a user.

The air cleaner A of this embodiment is configured so that the height L of the grip 91a of the handle 91 from the floor surface is 700 mm or less.

With such a configuration, the handle 91 can be easily gripped by a user, regardless of gender, without greatly bending down because an average height of dactylus points (vertical distance from a floor surface to a dactylus point of a middle finger when a human stands on the floor surface) of Japanese adults is about 662 mm as a whole (male: about 695 mm, female: about 632 mm).

Further, a depth S of the air cleaner A of this embodiment is about 300 mm.

With such a configuration, when the air cleaner A is laterally pushed and pulled, the air cleaner A is within a shoulder width of a human regardless of gender because an average shoulder width of Japanese adults is about 409 mm as a whole (male: about 426 mm, female: about 394 mm).

Thus, the air cleaner A can be easily moved even in a narrow passage or place.

Further, a width of a corridor in ordinary houses is about 850 mm to 900 mm, and thus when the air cleaner A is transversely placed on a corridor, a space of about 550 mm to 600 mm can be ensured.

Thus, even if the air cleaner A is placed on a corridor in ordinary houses, a sufficient space can be ensured through which a human or an object can pass.

Further, a width of a corridor in care facilities is about 1800 mm or larger, and thus when the air cleaner A is transversely placed on a corridor, a space of about 1500 mm can be ensured.

Thus, even if the air cleaner A is placed on a corridor in care facilities, a sufficient space can be ensured through which a human or an object can pass.

Also, a width of a generally used wheelchair is about 700 mm, and thus even if the air cleaner A is placed on a corridor in care facilities, wheelchairs can pass each other.

In the air cleaner A including the components configured as described above, the control means 47 controls the blowing fan 44 so that a flow rate of air passing through the deodorizing element 62 is a predetermined value or less, and thus controls the deodorizing element 62 to collect at least about 70% of an ammonia component or the like as an odor component in indoor air introduced into the air trunk R.

In this configuration, when the indoor air containing the ammonia component or the like taken in from the inlet 11 is discharged from the outlet 42 to the outside of the body, the indoor air from which about 70% or more of the ammonia component or the like is removed is released from the body into an indoor space.

Specifically, the air containing the ammonia component is simply once passed through the deodorizing element 62, and thus about 70% of the ammonia component can be removed from the indoor air taken in.

A rate of removal of an odor component contained in air by the deodorizing element 62 when air is once passed through the deodorizing element is referred to as a transient removal rate (one-pass removal rate) of an odor component.

For example, if no odor component is removed, the transient removal rate is 0% (the one-pass removal rate is 0%), and if 80% of an odor contained in air is removed, the transient removal rate is 80% (the one-pass removal rate is 80%).

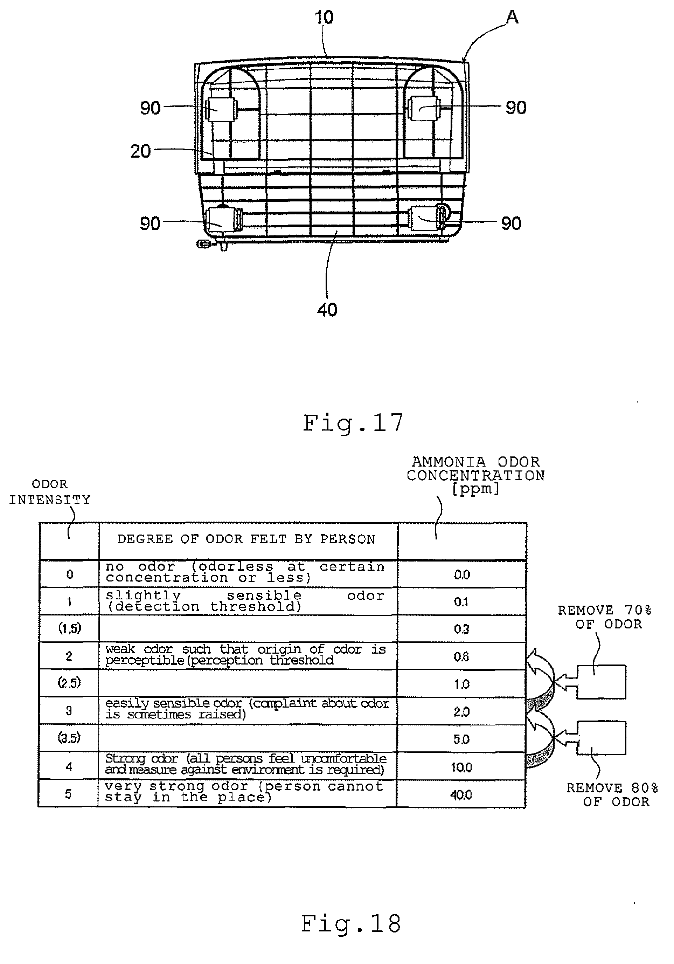

Now, with reference to Figure 18, odor intensity will be described. Figure 18 shows odor intensity in six grades corresponding to ammonia odor concentrations.

The odor intensity indicates, in six grades, a sense of odor measured using olfactometry (sensory test method) by an expert such as a licensed odor examiner, and is used as a basis for evaluation in setting reference values of 22 substances provided in the Offensive Odor Control Act. The odor intensity is a scale such that a person can feel a reduction in odor by sense of smell when there is one-grade reduction from a higher grade to a lower grade.

A person's sense of odor (degree of odor) in each grade of the odor intensity is as described below. Odor intensity 0 refers to no odor (odorless at a certain concentration or less). Odor intensity 1 refers to a slightly sensible odor (also referred to as a detection threshold). Odor intensity 2 refers to a weak odor such that an origin of the odor is perceptible (also referred to as a perception threshold). Odor intensity 3 refers to an easily sensible odor (a complaint about the odor is sometimes raised). Odor intensity 4 refers to a strong odor (all persons feel uncomfortable and a measure against the environment is required). Odor intensity 5 refers to a very strong odor (persons cannot stay in the place).

Correspondingly to the odor intensity, odor collecting capacities of a conventional air cleaner and the air cleaner of this embodiment will be described.

First, when a conventionally used portable air cleaner having a transient removal rate of odor of 50% is used in air with the odor intensity "4" (with an ammonia odor concentration of 10 ppm), air with an ammonia odor concentration of 5 ppm with 50% of odor being removed is released from the body.

Specifically, for the air cleaner with the transient removal rate of odor of 50%, air still containing a strong odor with the odor intensity not reaching "3" is released from the body into the indoor space.

Thus, for the air cleaner with the transient removal rate of odor of 50%, the air is returned into a room with the odor component being reduced but not reduced by one grade. Thus, the user cannot feel a reduction in odor from the released air.

Also, the air that is deodorized but whose reduction in odor cannot be felt by a person is blown out from the body into the indoor space. Thus, it takes time to deodorize the room, and also the odor spreads in the entire room.

Next, when the air cleaner of this embodiment that can be controlled to have a transient removal rate of odor of 80% is used in air with the odor intensity "4" (with an ammonia odor concentration of 10 ppm), air with an ammonia odor concentration of 2 ppm with 80% of an ammonia odor being removed from the air taken into the body is released from the body.

Specifically, for the air cleaner with the transient removal rate of odor of 80%, air with the odor intensity "3" that is one-grade reduced odor intensity is released from the body.

Thus, for the air cleaner with the transient removal rate of odor of 80%, the odor component is significantly reduced, and the air with the one-grade reduced odor intensity is released from the body into the room. Thus, the user can feel a reduction in odor from the released air.

Also, the air whose reduction in odor can be felt by the user is blown out from the body into the indoor space. Thus, the indoor air containing many odors can be more quickly attenuated, thereby allowing quicker deodorization in the room, and preventing the odor from spreading in the entire room.

The air cleaner controlled to have the transient removal rate of odor of 80% has been described. However, even if an air cleaner having a transient removal rate of odor of 70% is used in air with the odor intensity "3", the odor in the air released from the outlet 42 is reduced to the odor intensity "2" that is one-grade reduced odor intensity. Thus, the user can feel a deodorizing effect immediately after a start.

The air cleaner A is configured to be portable, and thus the size of the deodorizing element 62 is limited so as to be housed in a portable body. Specifically, unlike a stationary air cleaner provided in a predetermined place for use, the size (thickness) of the deodorizing element 62 cannot be simply increased to increase a deodorizing capacity.

Thus, in the air cleaner A, to maintain the transient removal rate of odor of 70% or more even for the deodorizing element 62 having the limited size as described above, the control means 47 controls the blowing fan 44 so that a flow rate of air containing an odor and passing through the deodorizing element 62 is about 0.15 m/s to about 1.8 m/s.

Thus, the wind speed is controlled to about 1.8 m/s or less, and thus the air cleaner that ensures portability while maintaining the transient removal rate of odor of 70% can be configured. Also, noise or vibration from the blowing fan or the like caused by an increase in the wind speed can be prevented.

The wind speed is set to about 0.15 m or more, thereby allowing a low operation in which cleaned air of 1 m³/min can be released from the body.

Next, in the air cleaner A, in a state where the blowing fan 44 is operating with the transient removal rate of odor of 70% or more, the control means 47 controls the blowing fan 44 so that a volume of indoor air passing through the air trunk R is about 10 m³/min or less.

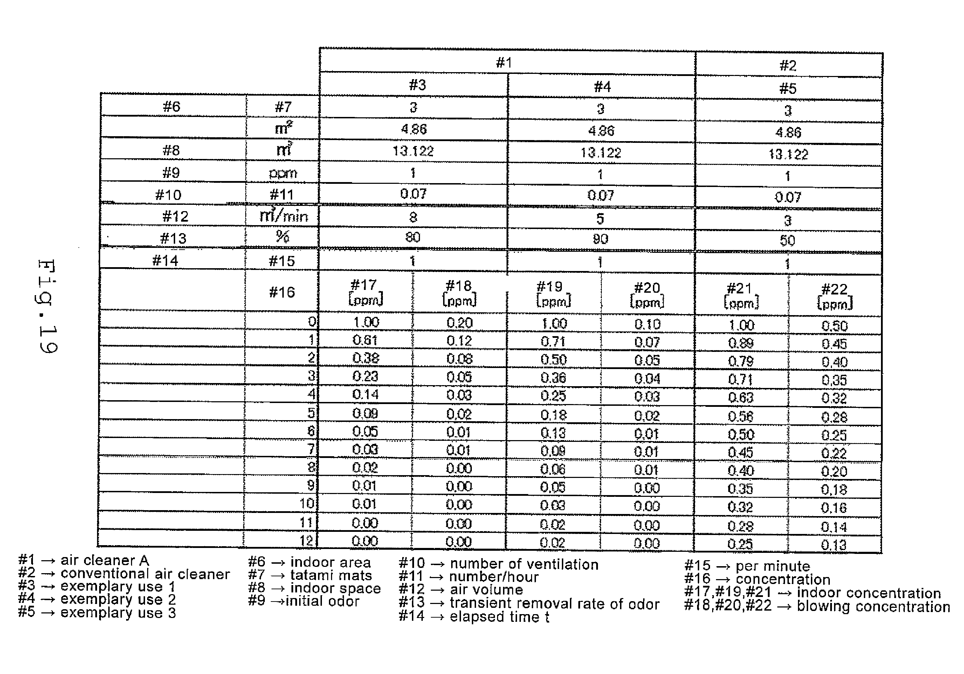

With reference to Figure 19 showing Exemplary uses 1 and 2, the air cleaner A thus controlled will be described. As a reference example, the conventional air cleaner (conventional example) will be also described.

First, a usage environment has an indoor area of three tatami mats (4.86 m²), an indoor space of 13.122 m³, and an initial ammonia odor concentration of 1 ppm. The usage environment is set assuming a space of one bed area partitioned by a curtain in a ward used by a plurality of people in hospitals or care facilities.

Specifically, a case is assumed where the air cleaner is conveyed from outside into a space of a predetermined volume partitioned by a structure such as a wall.

An elapsed time is a time that elapses from a start of use. An indoor concentration is a concentration of an ammonia odor contained in indoor air. A blowing concentration is a concentration of an ammonia odor contained in air released from the outlet 42 of the air cleaner A. A unit of the elapsed time is [min], and a unit of the indoor concentration and the blowing concentration is [ppm].

Changes of each odor concentration with elapsed time when each of the following air cleaners is used in such a condition will be described.

### (Exemplary use 1: transient removal rate of odor of about 80%, air volume per minute of 8 m³)

If the transient removal rate of odor is 80%, the odor intensity of air released from the outlet 42 is lower by one or more grades than the odor intensity of the indoor space, and thus the user can feel a reduction in odor by sense of smell from the start of use.

If the air volume per minute is 8 m³, the odor concentration of air released from the outlet 42 is reduced to 0.05 ppm that is one twentieth of the initial indoor odor concentration of 1 ppm in three minutes after the start of use. This is a value when the odor intensity is reduced by two grades.

Further, the ammonia odor concentration in the room is reduced to 0.05 ppm that is one twentieth in six minutes after the start of use. This value refers to an odor concentration in the case of substantially no odor.

As such, for Exemplary use 1, quick deodorizing to substantially no odor can be performed in 5 or 6 minutes after the start of use of the air cleaner A. Thus, work with odors performed around beds in hospitals or care facilities can be performed without diffusing the odors.

For example, diaper changing is taken as an example of such work for description. It usually takes 5 to 6 minutes to change a diaper of one person, which is within the time required for deodorizing as described above.

Specifically, the air cleaner A is used at the same time as diaper changing work between closing a curtain around a bed for starting diaper changing and opening the curtain after the changing. Thus, deodorizing of an odor caused by the diaper changing can be substantially finished at the time when the diaper changing work is finished.

Thus, in diaper changing in a ward used by a plurality of users, using the air cleaner A can prevent a flow of an odor from around a bed where the changing work is performed to other users.

Also, in facilities where diaper changing of many users is required, the diaper changing can be efficiently performed with little diffusion of an odor caused by the diaper changing.

### (Exemplary use 2: transient removal rate of odor of about 90%, air volume per minute of 5 m³)

In this exemplary use, an air volume is lower but a transient removal rate of odor is higher than that in Exemplary use 1.

If the transient removal rate of odor is 90%, the odor intensity of air released from the outlet 42 is lower by one or more grades than the odor intensity of the indoor space, and thus the user can feel a reduction in odor by sense of smell from the start of use.

If the air volume per minute is 5 m³, the odor concentration of air released from the outlet 42 is reduced to 0.05 ppm that is one twentieth of the initial indoor odor concentration of 1 ppm in 2 minutes after the start of use. This is a value when the odor intensity is reduced by two grades.

Further, the ammonia odor concentration in the room is reduced to 0.05 ppm that is one twentieth in 9 minutes after the start of use. This value refers to an odor concentration in the case of substantially no odor. As such, quick deodorizing to substantially no odor can be performed in 9 minutes after the start of use of the air cleaner A.

In this exemplary use, it takes longer time to reduce the ammonia odor concentration in the room to one twentieth than in Exemplary use 1, but the odor concentration of air released from the outlet 42 can be reduced to 0.05 ppm that is one twentieth of the initial indoor odor concentration in about 2 minutes after the start of use.

Also, the air cleaner A is operated with a reduced air volume, thereby reducing operation noise of the blowing fan 44 or the like, and reducing power consumption by the operation.

The exemplary use of the air cleaner A has been described above. For the portable air cleaner, the air volume is desirably controlled to 10 m³/min at a maximum.

This is because the air cleaner sized to be portable has a limited opening area of the outlet 42. Thus, increasing the volume of air released from the air cleaner A increases the speed of the air released from the outlet 42, which may raise dust.

Also, increasing the air volume increases the speed of air passing through the deodorizing element 62, which may reduce a transient removal rate of odor of the deodorizing element 62.

### (Conventional example: transient removal rate of odor of about 50%, air volume per minute of 3 m³)

Now, a reference exemplary use of a conventionally used air cleaner will be described for comparison.

Conventionally, there is a portable air cleaner having a transient removal rate of odor of about 50%.

For such an air cleaner, an odor concentration of air released from an outlet is only reduced to about 0.5 ppm. Thus, the odor intensity cannot be reduced by one grade, and it is difficult for the user to feel a reduction in odor by sense of smell at a start of use.

Also, the transient removal rate of odor is about 50% and the air volume per minute is 3 m³. Thus, an operation time of about 10 to 11 minutes is required between the start of use and when the user feels a reduction in odor in a room, that is, to reduce the odor concentration of 70% or more from the initial indoor odor concentration.

As such, it takes a long time to reduce the indoor odor, and thus it cannot be said that the conventional air cleaner is sufficient for use in diaper changing in hospitals or care facilities.

The air cleaner A described above in Exemplary uses 1 and 2 may be configured as described below. An air cleaner A conveyed from outside into a space of a predetermined volume partitioned by a structure such as a wall, including: a movable casing that includes an air trunk R having an inlet 11 and an outlet 42, and is arbitrarily accessible to a source of an odor containing an ammonia component generated in a lower level in the space; blowing means that is provided in the casing and blows indoor air introduced from the inlet 11 into the air trunk R from the outlet 42; a deodorizing element 62 that is located in the casing and through which the air introduced into the air trunk R passes; and control means that controls a blowing capacity of the blowing means 44, wherein the deodorizing element 62 includes a catalyst that adsorbs the ammonia component in the air introduced into the air trunk R and collects the ammonia component from the air, and in a state where the control means 47 controls an air volume of the blowing means 44 so that a flow rate per minute of the air passing through the deodorizing element 62 is one fifth or more of the volume of the space, the deodorizing element 62 collects the ammonia component in the indoor air introduced into the air trunk R at a predetermined rate or more in one passage of the air.

The air cleaner A is configured to be movable to near the source of the odor in the lower level in a room. Thus, the inlet 11 of the air cleaner A is arbitrarily accessible to the source of the odor, and an ammonia odor can be taken from the inlet 11 into the air cleaner A before diffusing in the space.

This configuration can quickly take into the air cleaner A an odor caused by diaper changing on a bed or the like provided in the room.

In addition to this, the control means controls the air volume of the blowing means so that the flow rate per minute of the air passing through the deodorizing element 62 is one fifth or more of the volume of the space partitioned by the structure such as the wall. This allows the odor in the indoor space to be collected in a short time (see Figure 19).

The lower level in the room refers to a range of 1 m or less from a floor surface in a space of a predetermined volume partitioned by a structure such as a wall, for example, a space in a ward or a space surrounded by a curtain.

In the air cleaner, it is preferable that the inlet 11 opens laterally of the casing, and a horizontal line passing through the center of the inlet 11 is in a range of 30 cm to 70 cm from the floor surface in the space.

The inlet 11 is thus formed, thereby allowing an odor from a bed that is a source of the odor in a ward or the like to be efficiently taken into the air cleaner A.

The air trunk R is bent upward on a downstream side of the inlet 11 in a front surface of the casing, thus the outlet 42 faces upward from a top surface of the casing, and the deodorizing element 62 is located between the inlet 11 and the bent portion in the air trunk R.

Thus, the air from which the odor is removed is blown upward of the casing. Thus, when the air cleaner A is located near the odor source for use, the air from which the odor is removed can be blown upward of the odor source.

This prevents disturbance of an airflow near the odor source, thereby preventing diffusion of the odor, and allowing the indoor air containing the odor to be efficiently taken into the air cleaner A.

### (Variant)

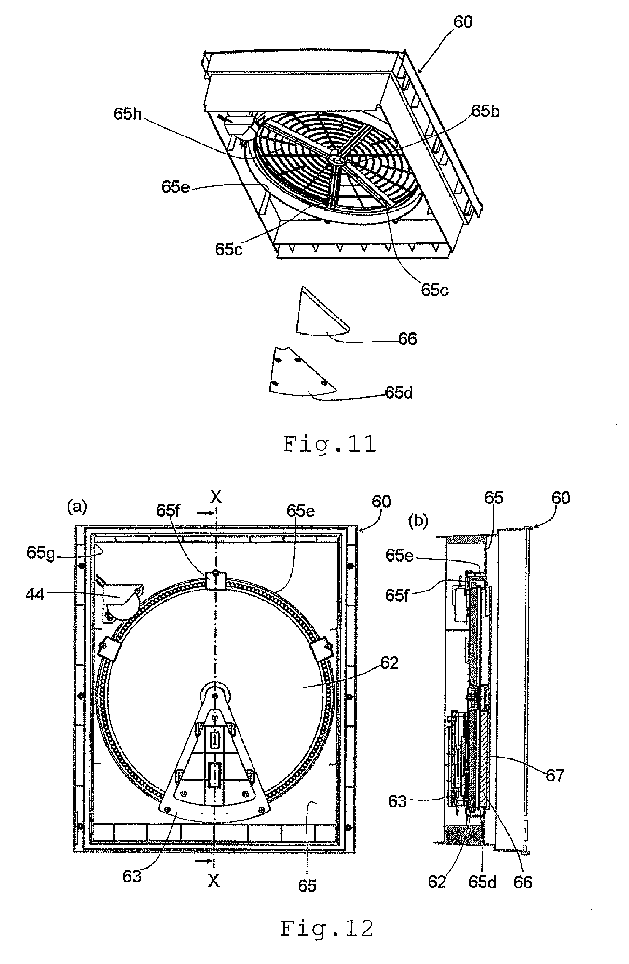

Next, with reference to Figures 11 and 12, a variant of the present invention will be described. The configurations as in Embodiment 1 are denoted by the same reference numerals, and descriptions thereof will be omitted.

A fan-like region that is located below a central support 65b at a center in an opening 65a in an intermediate partition plate 65 that constitutes a deodorizing portion 60, and formed with a predetermined open angle equally extending to the left and right around the central support 65b is covered with a substantially fan-like lid 65d from a back side of the intermediate partition plate 65.

The lid 65d is made of stainless, and secured to a beam 65c by a screw or the like from a rear side (back side) of the intermediate partition plate 65. The lid 65d is sized to cover a heater unit 63a of the heating unit 63 when facing the heater unit 63a.

With the lid 65d being mounted as described above, a heat insulating material 66 is provided in a part surrounded by a front surface of the lid 65d and the beam 65c. Further, a cover 67 is provided on a front side of the heat insulating material 66. The cover 67 is made of resin, and secured to the beam 65c by a screw or the like from the front side (surface side) of the intermediate partition plate 65.

With the configurations of the components as described above, the front surface of the lid 65d is covered with the heat insulating material 66. Thus, when the deodorizing element 62 is heated by the heating unit 63, heat in the heating space can be prevented from escaping from the surface of the lid 65d that faces the heating unit 63 to form a heating space.

Thus, the temperature of the heating space can be efficiently increased to allow efficient heating of the deodorizing element 62, and prevent burn injury because the user cannot directly touch the hot lid 65d.

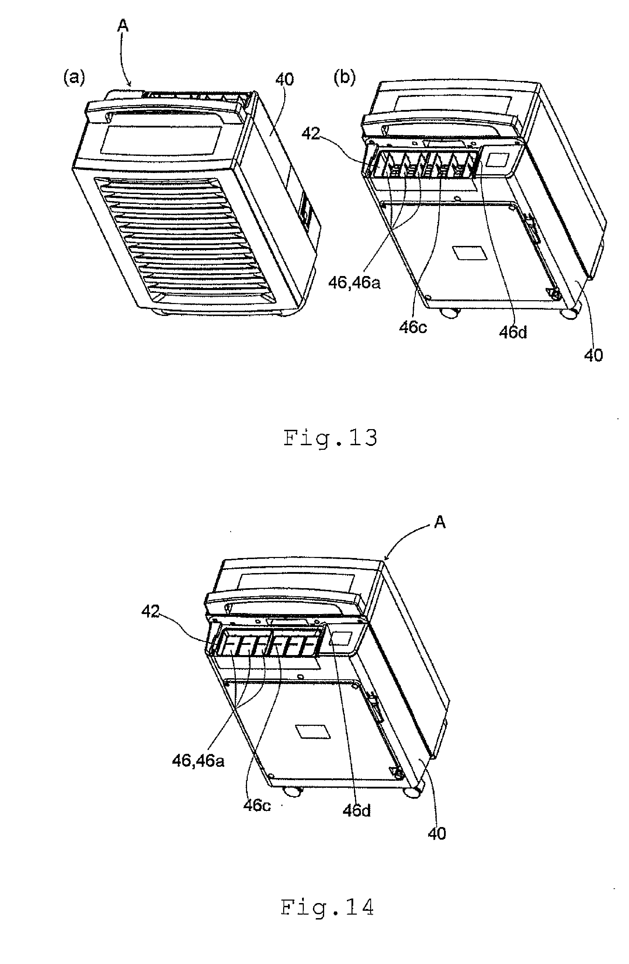

Next, with reference to Figures 13 and 14, another variant of the present invention will be described. The configurations as in Embodiment 1 and the above described variant are denoted by the same reference numerals, and descriptions thereof will be omitted.

In Figures 13 and 14, a grid, which is usually mounted to an opening of an outlet 42, is removed for describing movement of the louver 46.

First, the louver 46 includes, near the outlet 42, a plurality of plate-like wind direction plates 46a, a link mechanism 46c that connects the plurality of wind direction plates 46a and moves the wind direction plates 46a to a predetermined angle, and a drive portion (not shown) that drives the link mechanism 46c.

In the louver 46, the plurality of plate-like wind direction plates 46a are arranged in parallel at predetermined intervals on the opening of the outlet 42. Each wind direction plate 46a is journaled on the outlet 42 by shafts formed at opposite ends of the wind direction plate 46a.

A drive portion that drives the link mechanism 46c is connected to the control means 47 described later, and driven by a predetermined program depending on states of the air cleaner, thereby changing a direction of the louver 46.

Also, the wind direction plate 46a is sized to have a width in a fore/aft direction substantially matching a width of the outlet 42 in the fore/aft direction, and a maximum width in a lateral direction such that adjacent wind direction plates 46a block the outlet 42 when the louver 46 is closed, that is, when the wind direction plates 46a fall.

The control means 47 operates the drive portion that drives the link mechanism 46c so that the wind direction plates 46a of the louver 46 fall to block the outlet 42 during heating of the deodorizing element 62.

As such, the louver 46 blocks the outlet 42 during heating of the deodorizing element 62, thereby preventing an odor or the like generated from the deodorizing element 62 in heating from being released to the outside of the air cleaner A.

In this embodiment, the configuration in which the louver 46 provided in the outlet 42 blocks the outlet 42 has been described, but a shutter or the like that opens/closes the outlet 42 may be provided separately from the louver 46.

Not only in the outlet 42, but a shutter or the like that opens/closes an opening of the inlet 11 may be provided in the inlet 11. The inlet 11 is closed in heating of the deodorizing element 62, thereby preventing an odor or the like generated from the deodorizing element 62 in heating from being released to the outside of the air cleaner A.

- A: Air cleaner
- C: body case
- R: air trunk
- 10: front panel
- 11: air inlet
- 12: HEPA filter
- 13: prefilter
- 20: front case
- 21: frame
- 23: partition plate
- 26: operating portion
- 40: rear case
- 42: air outlet
- 44: blowing fan
- 45: partition plate
- 46: louver
- 46a: wind direction plate
- 46c: link mechanism
- 47: control means
- 60: deodorizing portion
- 61: frame
- 62: deodrizing element
- 63: heating unit
- 64: driving means
- 65: intermediate partition plate
- 65d: lid
- 66: heat insulating material
- 67: cover
- 90: wheel
- 91: handle
- 91a: grip

## Claims

1. An air cleaner having a deodorizing function comprising:
a portable casing that includes therein an air trunk having an inlet and an outlet;
blowing means that is provided in the casing and blows indoor air introduced from the inlet into the air trunk from the outlet;
a deodorizing element that is provided to interrupt the air trunk, and through which the air introduced into the air trunk passes; and
control means that controls an operation of the blowing means,
wherein the deodorizing element includes a catalyst that adsorbs an ammonia component in the air introduced into the air trunk and collects the ammonia component from the air, and
the control means controls the blowing means so that a flow rate of the air passing through the deodorizing element is a predetermined value or less, and thus the deodorizing element collects at least 70% of the ammonia component in the indoor air introduced into the air trunk.

2. The air cleaner having a deodorizing function according to claim 1, wherein during an operation of the blowing means, the control means controls the blowing means so that the flow rate of the air passing through the deodorizing element is 1.8 m/s or less.

3. The air cleaner having a deodorizing function according to claim 1, wherein during an operation of the blowing means, the control means controls the blowing means so that a volume of the indoor air passing through the air trunk is 10 m³/min or less.

4. The air cleaner having a deodorizing function according to claim 1, wherein the air trunk is horizontally formed in the casing, and
the deodorizing element is located upstream of a flow of the air introduced into the air trunk seen from a position of the outlet.

5. An air cleaner having a deodorizing function comprising:
a portable casing that includes therein an air trunk having an inlet and an outlet;
blowing means that is provided in the casing and blows indoor air introduced from the inlet into the air trunk from the outlet;
a deodorizing element that is provided to interrupt the air trunk, and through which the air introduced into the air trunk passes;
heating means that heats the deodorizing element; and
control means that controls an operation of the blowing means,
wherein the deodorizing element includes a catalyst that adsorbs an ammonia component in the air introduced into the air trunk and collects the ammonia component from the air,
the control means controls the blowing means so that a flow rate of the air passing through the deodorizing element is a predetermined value or less, and thus the deodorizing element collects 70% or more of the ammonia component in the indoor air introduced into the air trunk, and
the heating means has a capacity of heating, within a predetermined time, a predetermined portion of the deodorizing element to a predetermined temperature or more capable of recovering an ammonia component collecting capacity of the deodorizing element while the control means is not operating the blowing means.

6. The air cleaner having a deodorizing function according to claim 5, wherein the heating means has a capacity of heating the predetermined portion of the deodorizing element to a temperature of about 120°C or more within about 60 minutes.

7. The air cleaner having a deodorizing function according to claim 5, wherein the deodorizing element is configured so that the air from the blower passes from a first side surface toward a second side surface on an opposite side,
the heating means includes a heating element whose energization is controlled by the control means, and a heating space containing the heating element, and
the heating means is located close to at least one of the first side surface and the second side surface of the deodorizing element, and has, on a side facing the deodorizing element, an opening through which heat from the heating space is radiated.

8. An air cleaner having a deodorizing function comprising:
a portable casing that includes therein an air trunk having an inlet and an outlet;
blowing means that is provided in the casing and blows indoor air introduced from the inlet into the air trunk from the outlet;
a deodorizing element that is provided to interrupt the air trunk, and through which the air introduced into the air trunk passes; and
control means that controls an operation of the blowing means,
wherein the deodorizing element includes a catalyst that adsorbs an ammonia component in the air introduced into the air trunk and collects the ammonia component from the air, and
the control means controls the blowing means so that a flow rate of the air passing through the deodorizing element is a predetermined value or less, and thus the deodorizing element collects at least 70% of the ammonia component in the indoor air introduced into the air trunk, and so that a volume of the indoor air passing through the air trunk is 4 m³/min to 10 m³/min.

9. The air cleaner having a deodorizing function according to claim 8, wherein the control means controls the blowing means so that the flow rate of the air passing through the deodorizing element is 0.15 m/s to 1.8 m/s.

10. An air cleaner having a deodorizing function conveyed from outside into a space of a predetermined volume partitioned by a structure such as a wall, comprising:
a movable casing that includes therein an air trunk having an inlet and an outlet, and is arbitrarily accessible to a source of an odor containing an ammonia component generated in a lower level in the space;
electric blowing means that is provided in the casing and blows indoor air introduced from the inlet into the air trunk from the outlet;
a deodorizing element that is located in the casing and through which the air introduced into the air trunk passes; and
control means that controls a blowing capacity of the electric blowing means,
wherein the deodorizing element includes a catalyst that adsorbs the ammonia component in the air introduced into the air trunk and collects the ammonia component from the air, and
in a state where the control means controls an air volume of the electric blowing means so that a flow rate per minute of the air passing through the deodorizing element is one fifth or more of the volume of the space, the deodorizing element collects the ammonia component in the indoor air introduced into the air trunk at a predetermined rate or more in one passage of the air.

11. The air cleaner having a deodorizing function according to claim 10, wherein the lower level refers to a range of 1 m or less from a floor surface in the space, and
the inlet opens laterally of the casing, and a horizontal line passing through the center of the inlet is in a range of 30 cm to 70 cm from the floor surface in the space.

12. The air cleaner having a deodorizing function according to claim 11, wherein the air trunk is bent upward on a downstream side of the inlet in a front surface of the casing, thus the outlet faces upward from a top surface of the casing, and
the deodorizing element is located between the inlet and the bent portion in the air trunk.
